# EUROPEAN PATENT APPLICATION

(11) **EP 4 083 840 A1**
(43) Date of publication of application: **02.11.2022**
(21) Application number: 22167350.2
(22) Date of filing: 08.04.2022
(51) Int. Cl.: G06F 21/62, H04L 9/00

(54) **MEDICAL DATA AUTHENTICATION SYSTEM, MEDICAL DATA AUTHENTICATION METHOD, AND COMPUTER PROGRAM PRODUCT THEREOF**

(30) Priority: 27.04.2021 TW 110115210
(71) Applicant: AI Bioelectronic Healthtech Co., Ltd., 32450 Taoyuan City (TW)
(72) Inventor: HO, Yen-Yi, 32450 Taoyuan City (TW); HUANG, Yen-Yun, 32450 Taoyuan City (TW)
(74) Representative: Viering, Jentschura & Partner mbB Patent- und Rechtsanwälte

(57) **Abstract**

A medical data authentication system, a medical data authentication method, and a computer program product thereof are disclosed. The medical data authentication system includes a data establishment module, a signature module, a blockchain establishment module, and an authorization code generation module. The data establishment module is used for establishing a medical data. The signature module is used for receiving the medical data to obtain a medical digital signature. The blockchain establishment module is used for uploading the medical digital signature to a medical blockchain. The authorization code generation module is used for obtaining the medical digital signature from the medical blockchain to generate a corresponding authentication authorization code.

## Description

### BACKGROUND OF THE INVENTION

### 1. Field of the Invention

The present invention relates to a medical data authentication system, a medical data authentication method and a computer program product thereof; more particularly, the present invention relates to a medical data authentication system, a medical data authentication method and a computer program product thereof capable of authenticating related medical data and applying the same to blockchain related techniques.

### 2. Description of the Related Art

In modern medical care, if members of the general public want to obtain their own medical records or proof of vaccination, they usually obtain certificates issued by medical care facilities in the forms of hardcopy or softcopy. However, it is required that members of the general public submit an application to the medical care facilities in order to obtain the certificates, and this application process can be time consuming and expensive. Moreover, because a certificate individually issued by the medical care facility can easily be forged, it is very likely that other countries or government authorities will not accept such a certificate, which can be an obstacle to travelers who are visiting countries requiring proof of vaccination.

Therefore, there is a need to provide a medical data authentication system, a medical data authentication method and a computer program product thereof to mitigate and/or obviate the aforementioned problems.

### SUMMARY OF THE INVENTION

It is an object of the present invention to provide a medical data authentication system which is capable of authenticating related medical data and applying the same to blockchain related techniques.

It is another object of the present invention to provide a medical data authentication method for use in the abovementioned system.

It is yet another object of the present invention to provide a computer program product used for achieving the abovementioned method.

To achieve the abovementioned objects, the medical data authentication system of the present invention comprises a data establishment module, a signature module, a blockchain establishment module and an authorization code generation module. The data establishment module is used for establishing a medical data. The signature module is electrically connected to the data establishment module and is used for receiving the medical data to obtain a medical digital signature. The blockchain establishment module is electrically connected to the signature module and is used for uploading the medical digital signature to a medical blockchain. The authorization code generation module is used for obtaining the medical digital signature from the medical blockchain to generate a corresponding authentication authorization code.

The medical data authentication system of the present invention further comprises a scanning module, which is used for scanning an identification tag to read an identification code, and the data establishment module establishes the medical data in combination with the identification code.

The data establishment module of the medical data authentication system of the present invention further comprises establishing an identity identification module, such that the medical data is combined with the identity identification data to generate the authentication authorization code.

The medical data authentication system of the present invention further comprises a validation module, which is used for reading the authentication authorization code in order to validate the medical digital signature obtained from the medical blockchain.

The medical data authentication system of the present invention is applicable to authenticating vaccine data, and the medical data comprises a vaccine manufacturer data, a vaccine country of origin data, a vaccine manufacturing date data, a vaccine transportation temperature data or a vaccination record data.

The medical data authentication system of the present invention is applicable to authenticating a medical device, and the medical data comprises a medical device manufacturer data, a medical device country of origin data, a medical device manufacturing date data, a medical device transportation temperature data or a medical device usage record data.

The medical data authentication system of the present invention is applicable to authenticating a medicine, and the medical data comprises a medicine manufacturer data, a medicine country of origin data, a medicine manufacturing date data, a medicine transportation temperature data or a medicine usage record data.

The medical data authentication system of the present invention is applicable to authenticating a medical certificate document, and the medical data comprises a patient's basic information, a doctor's diagnosis record, a prescription record or a health checkup record.

A medical data authentication method of the present invention comprises the following steps: establishing a medical data; obtaining a medical digital signature according to the medical data; uploading the medical digital signature to a medical blockchain; and obtaining the medical digital signature from the medical blockchain to generate a corresponding authentication authorization code.

The medical data authentication method of the present invention comprises the step of scanning an identification tag to read an identification code so as to establish the medical data in combination with the identification code.

The medical data authentication method of the present invention comprises the step of uploading an identity identification data so that the medical data is combined with the identity identification data.

The medical data authentication method of the present invention comprises the step of reading the authentication authorization code in order to validate the medical digital signature obtained from the medical blockchain.

A computer program product of the present invention is stored in a computer readable storage medium for an electronic device to read and execute the medical data authentication method of the present invention.

Other objects, advantages, and novel features of the invention will become more apparent from the following detailed description when taken in conjunction with the accompanying drawings.

### BRIEF DESCRIPTION OF THE DRAWINGS

These and other objects and advantages of the present invention will become apparent from the following description of the accompanying drawings, which disclose several embodiments of the present invention. It is to be understood that the drawings are to be used for purposes of illustration only and not as a definition of the invention.

In the drawings, wherein similar reference numerals denote similar elements throughout the several views:
FIG. 1 illustrates a schematic drawing of a medical data authentication system according to the present invention.
FIG. 2 illustrates a flowchart of a medical data authentication method according to the present invention.

### DETAILED DESCRIPTION OF THE PREFERRED EMBODIMENT

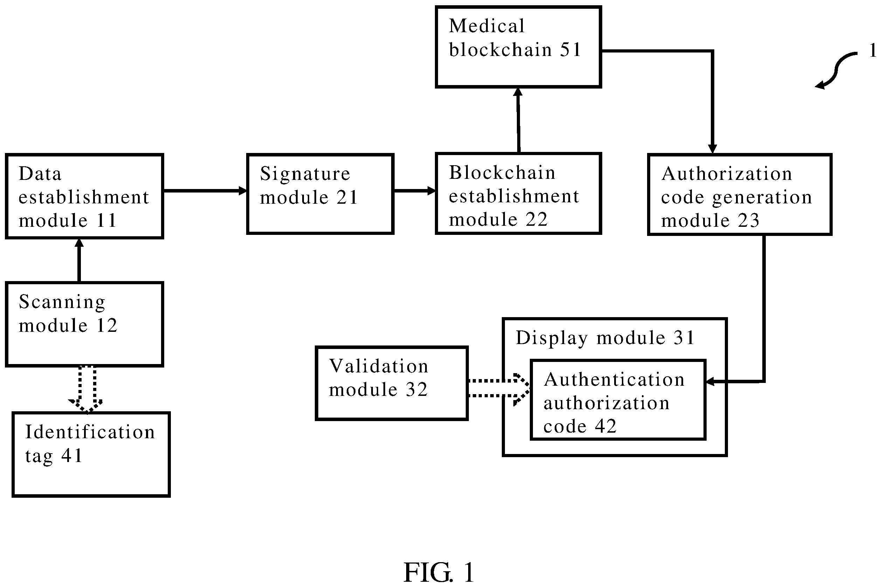

Please refer to FIG. 1, which illustrates a schematic drawing of a medical data authentication system according to the present invention.

In one embodiment of the present invention, the medical data authentication system 1 is applicable to a medical system, wherein each of the modules can be, but is not limited to being, separately installed on a computer host, a smart phone, a tablet computer or a wearable device at a data provider end, a data management end or a general user end. The medical data authentication system 1 can comprise a data establishment module 11, a scanning module 12, a signature module 21, a blockchain establishment module 22, an authorization code generation module 23 and a validation module 32. However, please note that the scope of the present invention is not limited to the abovementiond modules, and it is not necessary for the medical data authentication system 1 to include all of the abovementiond modules to achieve the object of the present invention.

The data establishment module 11 is located on the data provider end. The data provider end can be a healthcare personnel, a medical vendor or other related users. Therefore, the healthcare personnel, the medical vendor or the related user can input already-confirmed information via the data establishment module 11 to establish a medical data. The medical data of the present invention can be data such as vaccine data, medical device data, medicine data or medical certificate documents. In the case of vaccine data, the medical data comprises a vaccine manufacturer data, a vaccine country of origin data, a vaccine manufacturing date data, a vaccine transportation temperature data or a vaccination record data. In the case of medical device data, the medical data comprises a medical device manufacturer data, a medical device country of origin data, a medical device manufacturing date data, a medical device transportation temperature data or a medical device usage record data. In the case of medicine data, the medical data comprises a medicine manufacturer data, a medicine country of origin data, a medicine manufacturing date data, a medicine transportation temperature data or a medicine usage record data. In the case of medical certificate documents, the medical data comprises a patient's basic information, a doctor's diagnosis record, a prescription record or a health checkup record. Please note that the scope of the present invention is not limited to the abovementioned items.

The above medical care items such as vaccines, medical devices or medicines can be combined with identification tags 41. The identification tag 41 can be, but is not limited to, a radio frequency identification (RFID) tag, a one-dimensional barcode or a two-dimensional barcode. Different identification tags 41 have different identification codes. The scanning module 12 has a function of reading the tags. After the scanning module 12 scans the identification tag 41, the corresponding identification code will be transmitted to the data establishment module 11 for establishing the medical data in combination with the identification code. Please also note that the data establishment module 11 can not only combine the identification codes of the above medical care items but also establish an identity identification data for different users or patients, such that the medical data can be combined with the identity identification data of different users or patients.

The signature module 21 is electrically connected to the data establishment module 11 and is used for receiving the medical data so as to obtain a medical digital signature of the medical data by means of utilizing a digital signature algorithm (such as an RSA algorithm or a DSA algorithm) according to the key owned by the data provider end. Because the techniques of obtaining a digital signature by means of data calculations are well known to those skilled in the related art and are not the targeted improvement of the present invention, there is no need for further description. Each data provider end can utilize its own key to establish different medical digital signatures for different medical data. For example, a vaccine manufacturer may establish respective medical digital signatures for a vaccine manufacturer data, a vaccine country of origin data and a vaccine manufacturing date data; a logistic transportation end may establish a medical digital signature for a vaccine transportation temperature data; and a hospital may establish a medical digital signature for a vaccination record data.

The blockchain establishment module 22 is electrically connected to the signature module 21 and is used for uploading the medical digital signature to a trusted medical blockchain 51, which is the so-called on-chain procedure. A blockchain is a growing list of records (called blocks) that are linked together using cryptography. Each block contains a cryptographic hash of the previous block, a corresponding time stamp and transaction data. Such design makes blockchains resistant to modification of their data because once recorded, the data in any given block cannot be altered retroactively without altering all subsequent blocks. The blockchain technology utilizes the distributed ledger technology to allow both parties to effectively record a transaction, which can be validated permanently. As a result, this modification resistant characteristic of the blockchain technology ensures the accuracy of the medical digital signature.

The authorization code generation module 23 can be located on the data management end. The data management end can be a backend system which manages all established medical data, such as a hospital, a government authority such as the Ministry of Health and Welfare Social and Family Department, or a trusted third party. The blockchain establishment module 22 will upload all medical digital signatures to the medical blockchain 51, and the authorization code generation module 23 will obtain the uploaded medical digital signature from the medical blockchain 51 so as to generate a corresponding authentication authorization code 42. The authentication authorization code 42 can be in the form of digits, text or images, or it can be converted into a one-dimensional barcode or a two-dimensional barcode such as a QR code or other various forms without limiting the scope of the present invention. Because the data establishment module 11 can combine medical data with the identity identification data of different users or patients, the authorization code generation module 23 can integrate the medical digital signature having the same identity identification data to generate an authentication authorization code 42. As a result, the authorization code generation module 23 can display an authentication authorization code 42 exclusive to a specific user on a display module 31. The display module 31 can be equipped on a computer host, a smart phone, a tablet computer or a wearable device.

Finally, the validation module 32 can be used by general users, medical care facilities, customs bureaus or insurance companies for reading the authentication authorization code 42 so as to obtain the medical digital signature from the medical blockchain 51 for further validation. In the validation process, the validation module 32 firstly obtains the medical digital signature from the medical blockchain 51, and then the validation module 32 performs signature validation operations according to the corresponding key obtained from the data provider end. Further, the validation module 21 can return the medical digital signature to the signature module 21 of the data provider end in order to request the signature module 21 to verify the medical digital signature. Accordingly, the validation module 32 can validate the integrity and non-repudiation of the medical digital signature. For example, if the medical data is used for medical certificate documents, the insurance company can verify whether the medical digital signature generated from the medical data is modified or not via the validation module 32 so as to avoid insurance fraud due to modification of the medical data. Moreover, the validation module 32 can be used by medical care facilities or customs bureaus for verification purposes so as to further check if the medical digital signature is valid. In the case of a vaccine, for example, the validation module 32 can respectively check whether the medical digital signature obtained from the vaccine manufacturer or the medical digital signature obtained from the logistic transportation vendor is a valid or invalid signature. The validation module 32 can utilize a list or a diagram to display whether all medical digital signatures are valid. The presentation style of the validation module 32 of the present invention is not limited to the above description. Because the validation module 32 can only validate the integrity and non-repudiation of the medical digital signature without knowing detailed medical data from the medical digital signature, the validation can therefore be performed with the patient's privacy protected.

Please note that each of the modules of the medical data authentication system 1 can be in the form of a hardware device, a software program in combination with a hardware device, or firmware in combination with a hardware device. For example, a computer program can be stored in a computer readable storage medium for an electronic device to read and execute so as to achieve each function of the present invention. However, the scope of the present invention is not limited to the above mechanism. Further, the abovementioned embodiments only describe preferred embodiments of the present invention. To avoid redundant description, not all possible variations and combinations are described in detail in this specification. However, those skilled in the art will understand that not all the above modules or components are necessary parts, and also that, in order to implement the present invention, other more detailed known modules or components might also be included. It is possible that each module or component can be omitted or modified depending on different requirements, and it is also possible that other modules or components might be disposed between any two modules

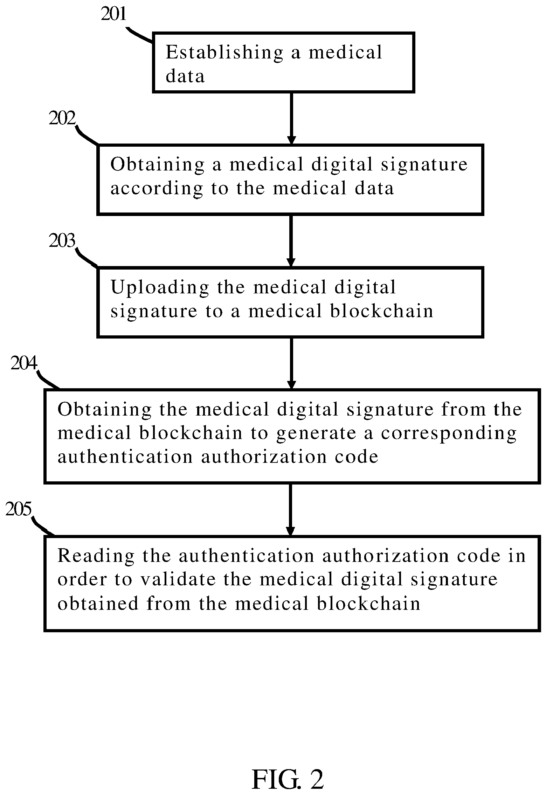

Now, please refer to FIG. 2, which illustrates a flowchart of a medical data authentication method according to the present invention. Please note that although the above medical data authentication system 1 is used as an example to describe the medical data authentication method of the present invention, the medical data authentication method of the present invention is not limited to implementation in the medical data authentication system 1 or other systems having the same structures.

First, the method performs step 201: establishing a medical data.

Acting as the data provider end, the healthcare personnel, the medical vendor or the related user can input a medical data via the data establishment module 11. The medical data of the present invention can be, but is not limited to, vaccine data, medical device data or medicine data. Meanwhile, the scanning module 12 can be used for reading the identification tag 41 of the medical care item such as a vaccine, medical device or medicine, and then for transmitting the obtained identification code to the data establishment module 11 for establishing the medical data in combination with the identification code. Alternatively, the medical data can be combined with identity identification data of different users or patients.

Then the method performs step 202: obtaining a medical digital signature according to the medical data.

The signature module 21 is used for receiving the medical data so as to obtain a medical digital signature of the medical data by means of utilizing a digital signature algorithm according to the key owned by the data provider end. Each data provider end can utilize its own key to establish different medical digital signatures for different medical data.

The method further performs step 203: uploading the medical digital signature to a medical blockchain.

Then the blockchain establishment module 22 is used for uploading the medical digital signature to a trusted medical blockchain 51, which is the so-called on-chain procedure.

Then the method performs step 204: obtaining the medical digital signature from the medical blockchain to generate a corresponding authentication authorization code.

The blockchain establishment module 22 will then upload all medical digital signatures to the medical blockchain 51, and the authorization code generation module 23 will obtain the uploaded medical digital signature from the medical blockchain 51 so as to generate a corresponding authentication authorization code 42. The authorization code generation module 23 can also integrate a medical digital signature having the same identity identification data to generate an authentication authorization code 42.

Finally, the method performs step 205: reading the authentication authorization code in order to validate the medical digital signature obtained from the medical blockchain.

The validation module 32 reads the authentication authorization code 42 so as to obtain the medical digital signature from the medical blockchain 51. Then the validation module 32 performs signature validation operations according to the corresponding key obtained from the data provider end, thereby validating the integrity and non-repudiation of the medical digital signature. The validation module 32 can also return the medical digital signature to the signature module 21 of the data provider end in order to request the signature module 21 to verify the medical digital signature.

Please note that the medical data authentication method of the present invention is not limited to performance according to the abovementioned orders and sequences. The orders and sequences of the abovementioned steps can be altered as long as the object of the present invention can be achieved.

According to the above description, the medical data authentication system 1 of the present invention can authenticate related medical data and therefore establish corresponding medical digital signatures; also, the related medical digital signatures can be stored in a blockchain to prevent arbitrary modification as well as to avoid direct exposure of medical data such that the accuracy of authentication content can be guaranteed and the patient's privacy can be protected.

Although the present invention has been explained in relation to its preferred embodiments, it is to be understood that many other possible modifications and variations can be made without departing from the spirit and scope of the invention as hereinafter claimed.

## Claims

1. A medical data authentication system (1), comprising:
a data establishment module (11), used for establishing a medical data;
a signature module (21), electrically connected to the data establishment module (11) and used for receiving the medical data to obtain a medical digital signature;
a blockchain establishment module (22), electrically connected to the signature module (21) and used for uploading the medical digital signature to a medical blockchain (51); and
an authorization code generation module (23), used for obtaining the medical digital signature from the medical blockchain (51) to generate a corresponding authentication authorization code (42).

2. The medical data authentication system (1) as claimed in claim 1, further comprising a scanning module (12) used for scanning an identification tag (41) to read an identification code, wherein the data establishment module (11) establishes the medical data in combination with the identification code.

3. The medical data authentication system (1) as claimed in claim 1, wherein the data establishment module (11) further comprises establishing an identity identification data and the medical data is combined with the identity identification data.

4. The medical data authentication system (1) as claimed in claim 1, further comprising a validation module (32) used for reading the authentication authorization code (42) so as to validate the medical digital signature obtained from the medical blockchain (51).

5. The medical data authentication system (1) as claimed in any of claims 1 to 4, wherein the medical data authentication system (1) is applicable to authenticating vaccine data, and the medical data comprises a vaccine manufacturer data, a vaccine country of origin data, a vaccine manufacturing date data, a vaccine transportation temperature data or a vaccination record data.

6. The medical data authentication system (1) as claimed in any of claims 1 to 4, wherein the medical data authentication system (1) is applicable to authenticating a medical device, and the medical data comprises a medical device manufacturer data, a medical device country of origin data, a medical device manufacturing date data, a medical device transportation temperature data or a medical device usage record data.

7. The medical data authentication system (1) as claimed in any of claims 1 to 4, wherein the medical data authentication system (1) is applicable to authenticating a medicine, and the medical data comprises a medicine manufacturer data, a medicine country of origin data, a medicine manufacturing date data, a medicine transportation temperature data or a medicine usage record data.

8. The medical data authentication system (1) as claimed in any of claims 1 to 4, wherein the medical data authentication system (1) is applicable to authenticating a medical certificate document, and the medical data comprises a patient's basic information, a doctor's diagnosis record, a prescription record or a health checkup record.

9. A medical data authentication method, applied in a medical data authentication system (1) for performing an authentication procedure, the method comprising the following steps:
establishing a medical data;
obtaining a medical digital signature according to the medical data;
uploading the medical digital signature to a medical blockchain (51); and
obtaining the medical digital signature from the medical blockchain (51) to generate a corresponding authentication authorization code (42).

10. The medical data authentication method as claimed in claim 9 further comprising the following step:
scanning an identification tag (41) to read an identification code so as to establish the medical data in combination with the identification code.

11. The medical data authentication method as claimed in claim 9 further comprising the following step:
uploading an identity identification data so that the medical data is combined with the identity identification data.

12. The medical data authentication method as claimed in claim 9 further comprising the following step:
reading the authentication authorization code (42) in order to validate the medical digital signature obtained from the medical blockchain (51).

13. A computer program product stored in a computer readable storage medium for an electronic device to read and execute the medical data authentication method as claimed in any of claims 9 to 12.
